# EUROPEAN PATENT APPLICATION

(11) **EP 2 494 920 A1**
(43) Date of publication of application: **05.09.2012**
(21) Application number: 11156507.3
(22) Date of filing: 02.03.2011
(51) Int. Cl.: A61B 5/145, G01N 33/487, G03B 21/53

(54) **Medical device capable of measuring a blood glucose level containing a data projector**

(71) Applicant: Sanofi-Aventis Deutschland GmbH, 65929 Frankfurt (DE)
(72) Inventor: The designation of the inventor has not yet been filed

(57) **Abstract**

A medical device (1) capable of measuring a blood glucose level comprises an optical display device (3). According to the invention, the display device (3) comprises a projector unit (3.2) with a light source and an array of optical elements (3.2.11). The projector unit (3.2) is capable of optically projecting and displaying an image (I) on a suitable surface (6) like, for example, a wall or a ceiling.

## Description

Medical device capable of measuring a blood glucose level

The invention relates to medical devices such as glucose meters capable of measuring a blood glucose level of a patient. The invention further relates to a method for using the medical device to measure a blood glucose level.

### Background of the Invention

Medical devices such as glucose meters capable of measuring the blood glucose level of a patient are known in the state of the art. These medical devices typically comprise a lancet or the like capable of taking or receiving a blood sample from a patient. The blood sample is brought into reaction with a test agent and analysed as to measure the blood glucose level by means of electrochemical or photometrical measurement methods. Nowadays glucose meters comprise a small and compact design that allows patients to carry the glucose meters with themselves in their everyday life. These medical devices are also capable of displaying measured test results on an optical screen only a few seconds after the blood sample has been taken.

In particular people suffering from diabetes are required to frequently measure their blood glucose levels and even may have to perform several blood glucose tests per day, i. e. before and/or after meals in order to estimate the proper dosage of a diabetes medicament that needs to be injected. On the one hand, the portable design of the glucose meter provides a certain degree of freedom in organizing the daily routine that should not be underestimated. However, the compact design of such medical devices inherently limits the size of the optical screen arranged thereon. Thus, people with limited eyesight may have difficulties to read the information displayed on the screen. This may result in an underdosage or overdosage of insulin or of an insulin derivate that is injected according to a misinterpreted value for the measured blood glucose level. It should be noticed that diabetes mellitus often causes an eye injury called diabetic retinopathy. Thus, visual impairment is common among people suffering from diabetes.

### Summary of the Invention

It is an object of the present invention to provide an improved medical device capable of measuring a blood glucose level that overcomes the limitations of the prior art devices.

It is a further object to provide a method for using the medical device to measure a blood glucose level so as to avoid an under- or overdosage of a medicament that is injected according to the measured blood glucose level.

The object is achieved by a medical device according to claim 1 and by a method for using a medical device according to claim 6.

Preferred embodiments of the invention are given in the dependent claims.

A medical device capable of measuring a blood glucose level comprises an optical display device. According to the invention, the display device comprises a projector unit with a light source and an array of optical elements. The projector unit is capable of optically projecting and displaying an image on a suitable surface like, for example, a wall or a ceiling. The projector unit avoids limitations to font size inherent to displays arranged on a housing of the medical device. Thus, the projector unit allows for displaying relevant measured quantities in a size large enough to be easily read even by persons with limited eyesight. A reliable diagnosis may thus be based on the measurement results. In particular, the display of information via the projector unit helps to properly dose a medicament according to the measured blood glucose level.

The medical device with the projector unit is particularly suited for patients suffering from diabetes that have to adjust the dose of the medicament according to their current blood glucose level. However, it is foreseen to equip other portable medical devices capable of measuring medical parameters to improve the readability of measured results.

Preferably, the medical device comprises a trigger element like a push button that is actuatable by a user, wherein manual actuation of the trigger element projects the image onto the surface for better readability of the measured values. The trigger element allows for an optional activation of the projector unit when needed.

The array of optical elements that may be adjustable to focus the image by manually operating an adjustment element to improve the readability of the information contained in the projected image.

Additionally or alternatively, the focus of the image may be provided by an autofocus function of the medical device. The projector unit may comprise at least one optical sensor, a control unit and a drive capable of automatically adjusting the array of optical elements to focus the image. The autofocus function is particular suited for users with limited vision that have problems adjusting the focus.

According to one possible embodiment of the invention, the light source of the medical device comprises at least one light emitting diode (LED). Light emitting diodes have compact dimensions and avoid generate unwanted heat during use and thus are particularly suited to used in portable medical device.

According to an alternative embodiment, the light source is provided by a low energy laser. Low energy laser inherently provide focused images, so that an adjustment of the array of optical elements may be omitted.

A method for using a medical device capable of measuring and monitoring a blood glucose level comprises the steps of providing a blood sample, introducing the blood sample into the medical device, measuring the glucose level of the blood sample and actuating a trigger element to optically project an image containing information about the measured glucose level onto a suitable surface. The method avoids a misinterpretation of the displayed information even if the performed by a person suffering from a visual impairment.

Accordingly, the method may further comprise the step of focusing the image either automatically by the means providing the autofocus function or manually operating the adjustment element to provide a better readability of the information projected onto the exterior surface.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows schematically the medical device with a projector unit in a plan view and
- Figure 2: shows schematically a section of the medical device with the projector unit in a perspective view.
- Figure 3: shows schematically components of the projection unit.

Corresponding parts are marked with the same reference symbols in all figures.

### Detailed Description of Preferred Embodiments

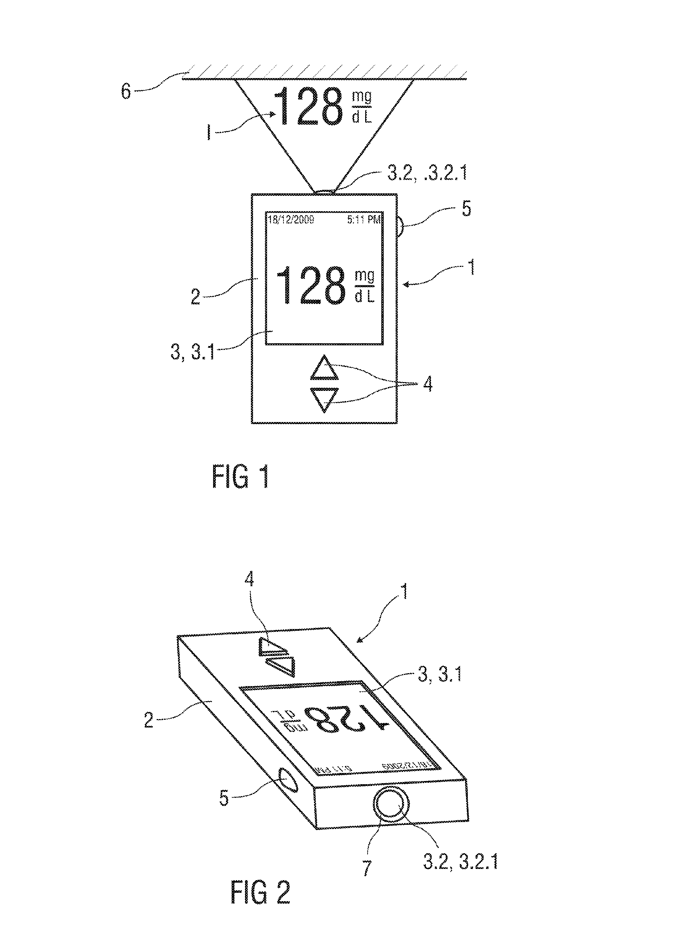

Figure 1 shows a medical device 1 capable of measuring a blood glucose level of a patient in a plan view. The medical device 1 may comprise a lancet or the like capable of taking a blood sample of the patient. Alternatively, the medical device 1 may comprise a suitable means to introduce the blood sample to the medical device 1. The medical device 1 further comprises suitable analysing means capable of bringing the blood sample into reaction with a test agent and analyzing the blood sample by photometric or electrochemical methods to determine the current blood glucose level of the patient.

The medical device 1 is a portable device with a compact housing 2 and comprises an optical display device 3 for displaying relevant information and in particular the measured blood glucose level. The displayed information may include the time and date of the measurement. The optical display device 3 has a screen 3.1 such as a liquid crystal display (LCD) that is arranged on the housing 2. Thus, the size of the screen 3.1 is limited the overall dimensions of the housing 2.

A plurality of control elements 4 is arranged on the housing 2 that allows a user of the medical device 1 to change various display properties such as brightness, contrast, the font size or the system of units used for representing the measured blood glucose level. In an example embodiment, the system of units may be interchanged between millimoles per litre (mmol/l) and milligrams per decilitre (mg/dl), for example by a user input or in accordance with a language or country setting. In an alternative embodiment, setting the system of units is prevented because of regulatory requirements

The optical display device 3 further comprises a projector unit 3.2 that is, upon manual actuation of a trigger element 5, capable of optically projecting an image I of the information onto a suitable exterior surface 6 such as a wall or a ceiling. The projector unit 3.2 is adapted to display the relevant information and in particular the measured values of the blood glucose level onto the surface 6 in a manner that allows the information to be read even by people with limited vision.

In an alternative embodiment of the invention, the screen 3.1 is omitted and the relevant information is solely displayed via the projector unit 3.2.

Preferably, the control elements 4 can be manually operated to alter display properties of the projected image I.

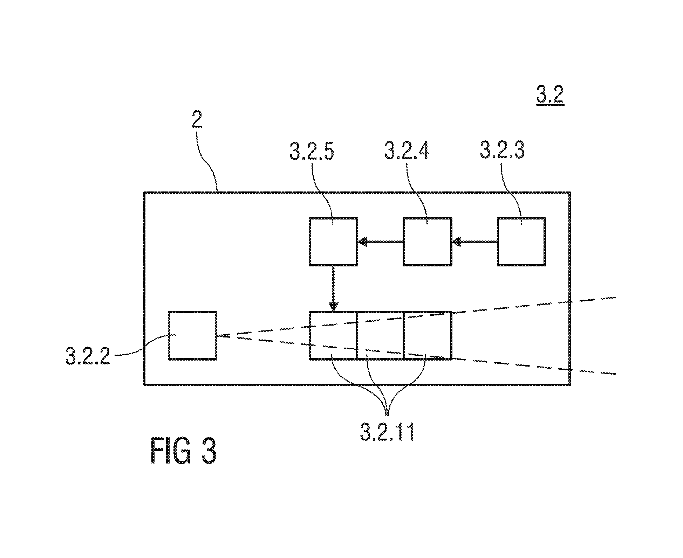

The components of the projector unit 3.2 are shown in more detail in figure 3.

The projector unit 3.2 comprises a light source 3.2.2 disposed within the housing 2 and an array of optical elements 3.2.11 comprising at least one lens 3.2.1. The light source 3.2.2 may comprise at least one light emitting diode (LED) or, alternatively, a low energy laser.

The array of optical elements 3.2.11 is adjustable to focus an image I displaying the information in particular to improve readability of the displayed information.

Figure 2 shows a section of the medical device 1 with the projector unit 3.2 in a perspective view. A ring shaped adjustment element 7 is arranged around the lens 3.2.1 that may be manually operated to focus the projected image I.

Additionally or alternatively, the medical device 1 may comprise means that automatically focuses the projected image. The autofocus feature may be provided by at least one optical sensor 3.2.3 capable of detecting a current focus, a control unit 3.2.4 and a drive unit 3.2.5 adapted to adjust the array of optical elements 3.2.11 for focusing the projected image I.

A method of using the medical device 1 for determining a current blood glucose level comprises the steps of obtaining a blood sample, introducing the blood sample into the medical device 1 for measurement of the blood glucose level in the blood sample, pointing the medical device 1 onto a suitable exterior surface 6 and actuating the trigger element 5 to optically project the image l containing information about the measured glucose level onto surface 6.

Additionally, the focus of the projected image I displaying the relevant information may be adjusted for better readability.

Figure 3 show schematically the components of the projector unit 3.2 comprising the light source 3.2.2, the array of optical elements 3.2.11, the optical sensor 3.2.3, the control unit 3.2.4 and the drive unit 3.2.5 arranged within the housing 2 of the medical device 1.

### List of References

- 1: medical device
- 2.: housing
- 3: display device
- 3.1: screen
- 3.2: projector unit
- 3.2.1: lens
- 3.2.11: optical element
- 3.2.2: light source
- 3.2.3: optical sensor
- 3.2.4: control unit
- 3.2.5: drive unit
- 4: control element
- 5: trigger element
- 6: surface
- 7: adjustment element
- I: image

## Claims

1. Medical device (1) capable of measuring a blood glucose level comprising an optical display device (3), **characterized in that** the display device (3) comprises a projector unit (3.1) with a light source and an array of optical elements (3.2.11), wherein the projector unit (3.1) is capable of optically projecting and displaying an image (I) on a suitable surface (6).

2. Medical device (1) according to claim 1, **characterized in that** the image (I) is projectable onto the surface (6) upon actuation of a trigger element (5).

3. Medical device (1) according to claim 1 or 2, **characterized in that** the array of optical elements (3.2.11) is adjustable to focus the image (I) by manually operating an adjustment element (7).

4. Medical device (1) according to one of the previous claims, **characterized in that** the projector unit (3.2) comprises at least one optical sensor (3.2.3), a control unit (3.2.4) and a drive unit (3.2.5) capable of automatically adjusting the array of optical elements (3.2.11) to focus the image (I).

5. Medical device (1) according to one of the previous claims, **characterized in that** the light source comprises at least one light emitting diode.

6. Medical device (1) according to one of the previous claims, **characterized in that** the light source is provided by a low energy laser.

7. Method for using a medical device (1) capable of measuring and monitoring a blood glucose level, comprising the steps of providing a blood sample, introducing the blood sample into the medical device (1), measuring the glucose level of the blood sample, actuating a trigger element (7) to optically project an image (I) containing information about the measured glucose level onto a suitable surface (6).

8. Method according to claim 7 further comprising the step of focusing the projected image (l).
